# EUROPEAN PATENT APPLICATION

(11) **EP 4 393 531 A1**
(43) Date of publication of application: **03.07.2024**
(21) Application number: 22887295.8
(22) Date of filing: 20.07.2022
(51) Int. Cl.: A61M 5/44, A61M 5/28, A61M 5/315, A61M 5/20, A61M 5/42

(54) **SMART INSULIN INJECTION DEVICE CAPABLE OF CONSTANT-TEMPERATURE STORAGE**

(30) Priority: 01.11.2021 KR 20210147592; 01.11.2021 KR 20210147593
(71) Applicant: Mcure Co. Ltd., Wonju-si, Gangwon-do 26359 (KR)
(72) Inventor: LEE, Man Kyu, Seongnam-si Gyeonggi-do 13525 (KR); KWAK, Ho Jung, Wonju-si Gangwon-do 26380 (KR); LEE, Suk Chan, Seongnam-si Gyeonggi-do 13531 (KR)
(74) Representative: BCKIP Part mbB
(86) International application number: PCT/KR2022/010574
(87) International publication number: WO 2023/075089

(57) **Abstract**

The invention relating to a thermostatically-storable smart insulin injection device is configured to include: a casing, a syringe installed inside the casing and with a guide installed at a lower end to be in contact with the skin during the insulin injection; a cartridge housed inside the syringe in a state of insulin being stored and with a piston installed at an upper side and with a needle surrounded by the guide provided at a lower side; an insulin injection unit pushing the piston of the cartridge to inject the insulin into the skin through the needle; and a Peltier module supplying cold or warm air to the syringe to maintain temperature of the interior of the syringe with the cartridge housed inside the syringe within a set temperature range.

## Description

### Technical Field

The invention relates to an insulin injection device, and more particularly, to an insulin pen-type thermostatically-storable smart insulin injection device capable of storing the insulin at a constant temperature.

### Background

Diabetes mellitus is a chronic disease disorder occurring when glucose (blood sugar) in the blood of a patient is higher in concentration than that of normal people due to a deficiency of insulin in the body. And thus, most patients need to regularly administer insulin, a hypoglycemic agent.

The insulin syringes or the insulin pens are commonly used to administer the insulin.

Herein, the insulin syringes are usually used on a disposable basis, as the user (patient) injects the insulin stored in the syringe by pushing the piston of the syringe after the needle of the syringe is directly punctured into the skin, and then the syringe is discarded.

Therefore, the insulin pen is then attached to an auto-injection device with a cartridge storing a quantity of the insulin that can be injected multiple times. After the user (patient) punctures the needle directly into the skin, the user presses a button on the auto-injection device so that the insulin is automatically injected into the skin.

Since such an insulin pens store the insulin that can be used multiple times in the cartridge, after one injection of the insulin, the remaining insulin needs to be stored in a thermostatic state to prevent deterioration of the insulin.

However, since the insulin pen does not have a structure that can maintain the thermostatic state, the remaining insulin is generally stored in a separate storage device that can maintain the thermostatic state. Therefore, the storage of the insulin is much more troublesome and difficult to carry around.

### Detailed Description of the Invention

### Technical Problem

The invention is conceived to solve the problems of the afore-mentioned related art, and is intended to provide an insulin pen-type thermostatically-storable smart insulin injection device that can store the insulin stored in a cartridge at a constant temperature, capable of preventing the deterioration of the insulin.

### Solution to Problem

A thermostatically-storable smart insulin injection device according to the invention to solve the afore-mentioned problems is configured to include: a casing; a syringe installed inside the casing and with a guide installed at a lower end to be in contact with the skin during the insulin injection; a cartridge housed inside the syringe in a state of being stored and with a piston installed at an upper side and with a needle surrounded by the guide provided at a lower side; an insulin injection unit pushing the piston of the cartridge to inject the insulin into the skin through the needle; and a Peltier module supplying cold or warm air to the syringe to maintain temperature of the interior of the syringe with the cartridge housed inside the syringe within a set temperature range.

Herein, the insulin injection unit is configured with: a drive motor; a screw shaft connected to a motor shaft of the drive motor and rotating with the motor shaft; and a piston pressing block connected to the screw shaft to push the piston of the cartridge by moving forward in the syringe.

And the Peltier module is configured with: a temperature sensor measuring the temperature of the syringe; a Peltier element generating the cold or warm air according to a value measured by the temperature sensor; a conducting plate facing one side surface of the Peltier element and being in contact with the syringe to transfer the cold or warm air; a heat sink plate facing the other side surface of the Peltier element; and a blower fan providing airflow to the heat sink plate.

In addition, the Peltier module is configured to further include a suction unit that, when the guide is in contact with the skin, sucks the air between the cartridge and the syringe so that the skin is pulled up, to allow the skin to be automatically punctured by a needle.

In addition, the suction tube with one side end being connected to a space between the cartridge and the syringe; a vacuum pump being connected to the other side end of the suction tube to suck air in the space between the cartridge and the syringe; and a pressure sensor installed on the conduit of the suction tube or at the end of the suction tube to measure the pressure of the air being sucked.

The thermostatically-storable smart insulin injection device further includes a control unit electrically connected to the insulin injection unit, the Peltier module, and the suction unit. The control unit displays information about the insulin injection amount, the number of the insulin injections and the temperature of the syringe and transmits the information to a smartphone connected through a communication network.

In addition, when the temperature of the syringe is outside the set temperature range, the control unit commands the control unit so as to operate the Peltier module through the smartphone.

At an upper end of the casing, an end cap is installed with a number of air discharge holes along the edge to discharge the air sucked by the suction unit to the outside.

In addition, a battery that supplies electricity to the suction unit and the insulin injection unit is installed inside the casing, and at the end cap, the charging holes are formed so as to supply electricity to the battery to allow the electricity to be stored.

### Advantageous Effects of Invention

The thermostatically-storable smart insulin injection device according to the invention can allow the temperature of the syringe housed in the cartridge to be maintained at a constant temperature by operation of the Peltier module, so that the insulin remaining in the cartridge after the insulin injection can be stored in a thermostatic state, and thus, this has an advantage that the insulin can be stored for a long period of time without deterioration of the insulin.

In addition, after the user allows the guide to be in contact with the skin, air is sucked by the suction unit to form a vacuum, and the skin is lifted upward, so that the skin is automatically punctured by the needle. And once the skin is punctured by the needle, the insulin injection unit is automatically operated to automatically inject the insulin into the skin, and thus, this has an effect of reducing fear and refusal feeling of the insulin injection.

In addition, The thermostatically-storable smart insulin injection device according to the invention has advantages that various information about the insulin injection amount, the number of times of insulin injection, and the temperature of the syringe is supplied through the control unit, so that the state of the insulin injection device can be checked in real time, and even when the insulin injection device does not immediately exist nearby, the Peltier module can be operated by operation of the mobile phone, so that the temperature of the syringe can be adjusted remotely.

### Brief Description of Drawings

Figs. 1 to 6 are diagrams illustrating a thermostatically-storable smart insulin injection device according to the invention;
Fig. 7 is a cross-sectional diagram illustrating the thermostatically-storable smart insulin injection device according to the invention;
Fig. 8 is a diagram illustrating a mode where cold or warm air is transferred from a Peltier element of the thermostatically-storable smart insulin injection device according to the invention to a syringe;
Fig. 9 is a diagram illustrating a mode where a suction unit of the thermostatically-storable smart insulin injection device according to the invention is operated to suck air;
Fig. 10 is a diagram illustrating a mode where an insulin injection unit of the thermostatically-storable smart insulin injection device according to the invention is operated;
Fig. 11 is a diagram illustrating a mode where the thermostatically-storable smart insulin injection device according to the invention is operated to allow skin to be lifted up, so that insulin is injected and, after that, a needle is inserted; and
Fig. 12 is a diagram illustrating a cartridge of the thermostatically-storable smart insulin injection device according to the invention.

### Description of Embodiments

A thermostatically-storable smart insulin injection device according to the invention to solve the afore-mentioned problems is configured to include: a casing; a syringe installed inside the casing with a guide installed at a lower end to be in contact with the skin during the insulin injection; a cartridge housed inside the syringe in a state of being stored and with a piston installed at an upper side and with a needle surrounded by the guide provided at a lower side; an insulin injection unit pushing the piston of the cartridge to inject the insulin into the skin through the needle; and a Peltier module supplying cold or warm air to the syringe to maintain temperature of the interior of the syringe with the cartridge housed inside the syringe within a set temperature range.

### MODE FOR CARRYING OUT THE INVENTION

Hereinafter, an example of a thermostatically-storable smart insulin injection device according to the invention will be described in detail with reference to the accompanying drawings.

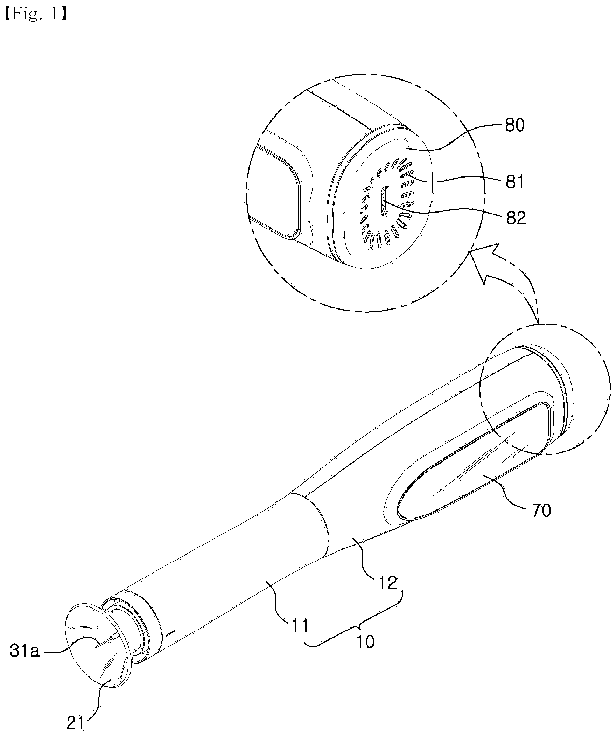

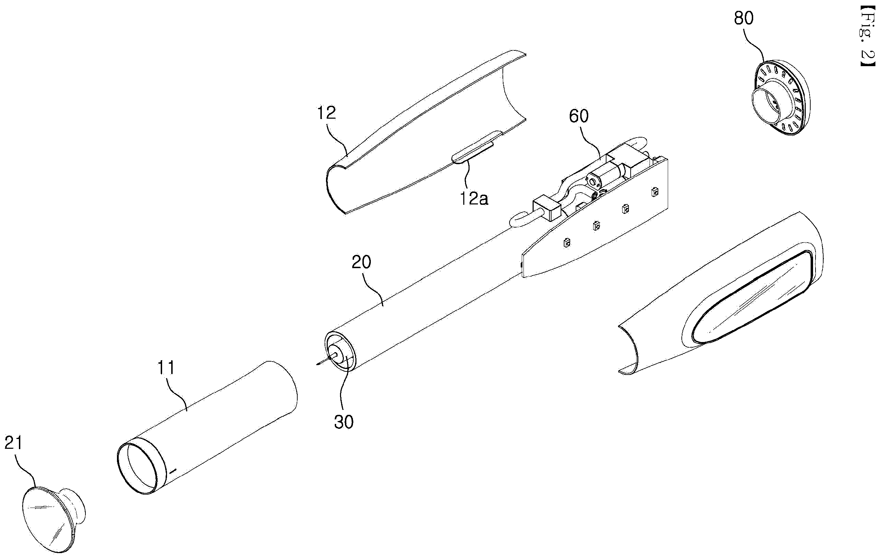

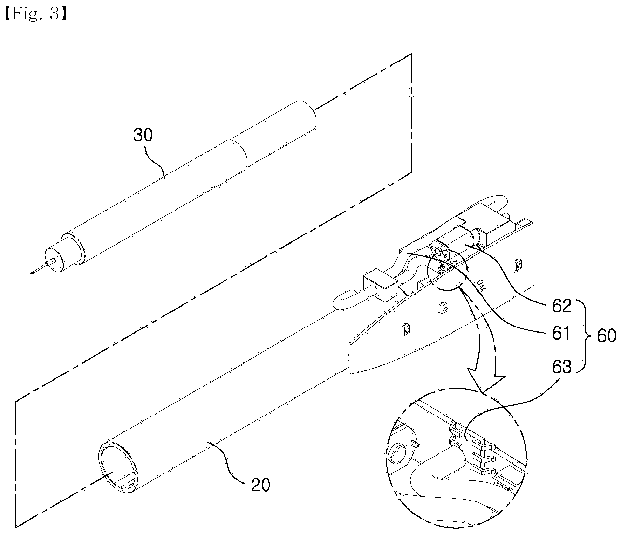

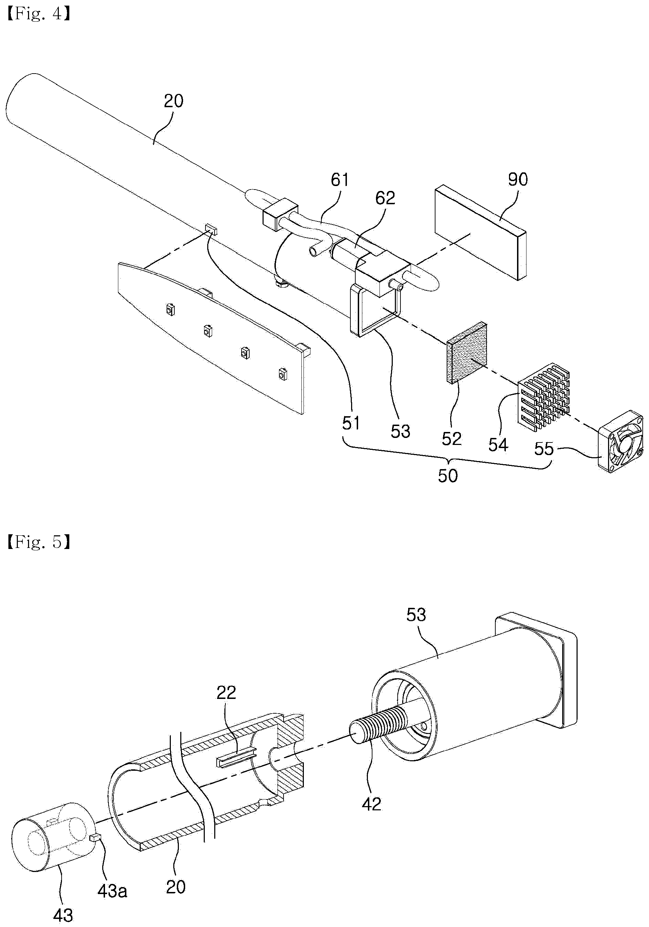

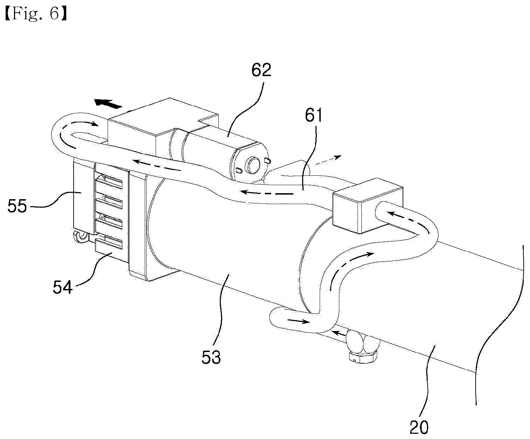

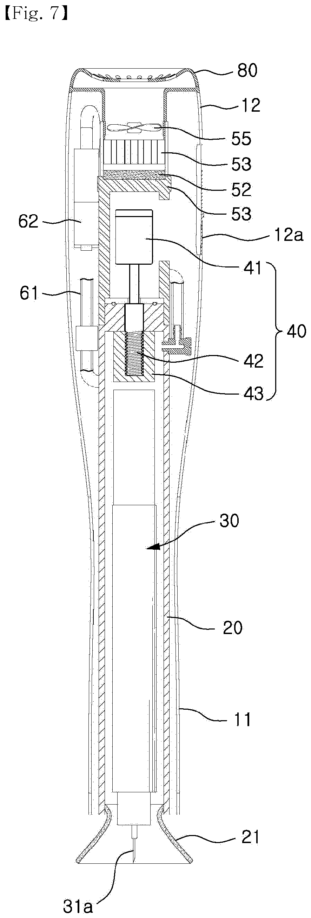

Figs. 1 to 6 are diagrams illustrating a thermostatically-storable smart insulin injection device according to the invention. Fig. 7 is a cross-sectional diagram illustrating the thermostatically-storable smart insulin injection device according to the invention.

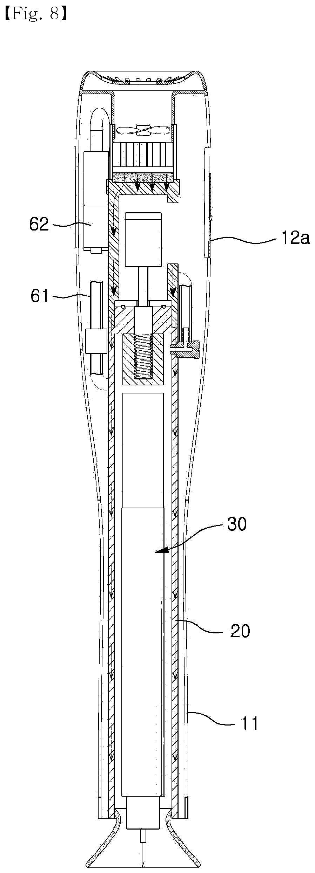

Fig. 8 is a diagram illustrating a mode where cold or warm air is transferred from the Peltier element of the thermostatically-storable smart insulin injection device according to the invention to the syringe.

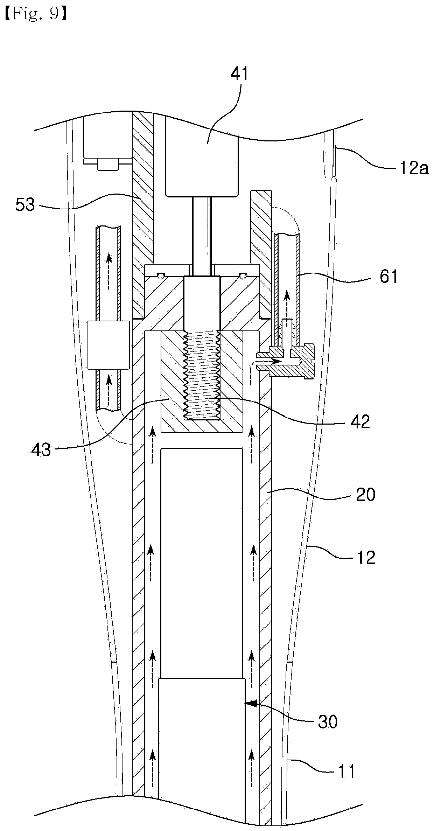

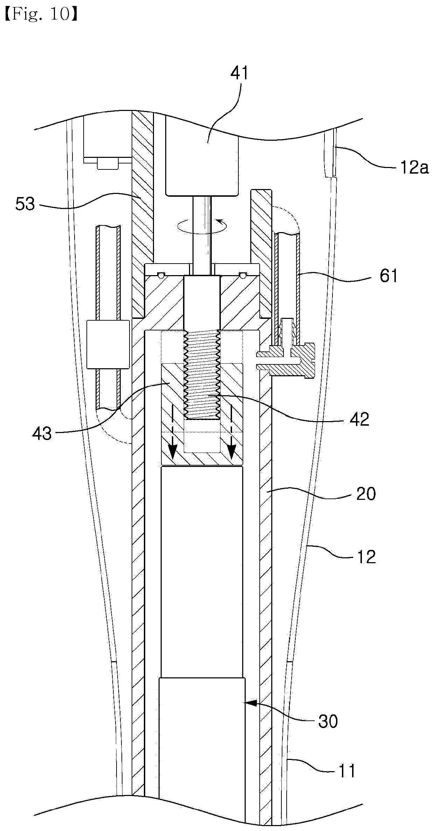

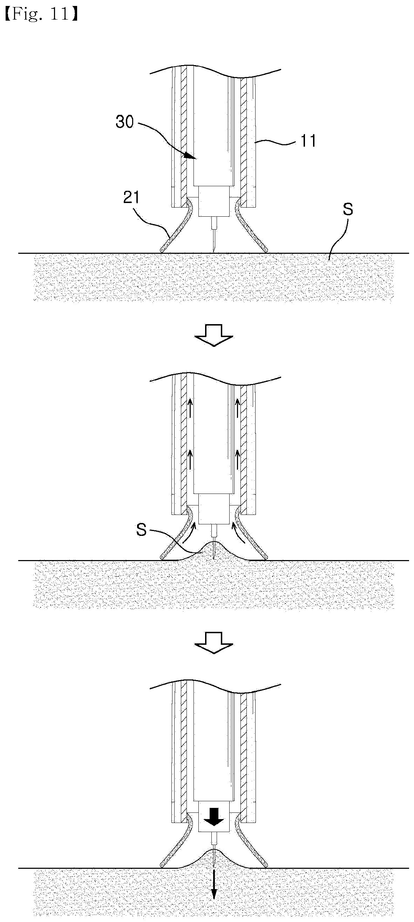

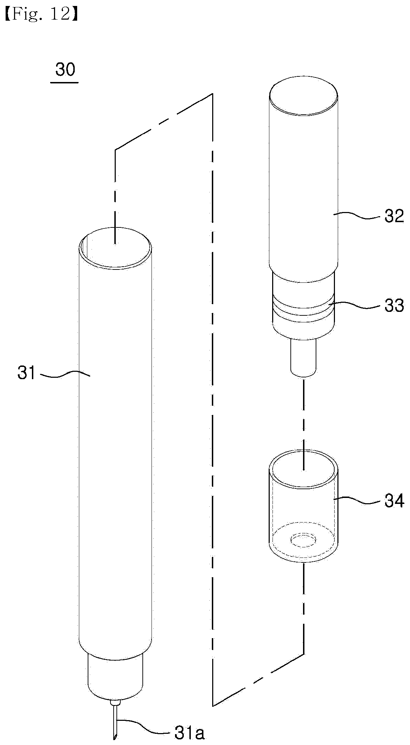

Fig. 9 is a diagram illustrating a mode where the suction unit of the thermostatically-storable smart insulin injection device according to the invention being operated to draw air. Fig. 10 is a diagram illustrating a mode where the insulin injection unit of the thermostatically-storable smart insulin injection device according to the invention being operated. Fig. 11 is a diagram illustrating a mode where thermostatically-storable smart insulin injection device is operated and skin is lifted up and the insulin is injected after the skin is punctured. Fig. 12 is a diagram illustrating a mode where a cartridge of a thermostatically-storable smart insulin injection device according to the invention. The thermostatically-storable smart insulin injection device according to the invention is configured to include: a casing 10; a syringe 20 installed inside the casing 10; a cartridge 30 with the insulin being stored inside; an insulin injection unit 40 injecting the insulin stored in the cartridge 30 into the skin S; a Peltier module 50 supplying the cold or warm air to the syringe 20; a suction unit 60 generating a vacuum between the syringe 20 and the cartridge 30; a control unit 70 provided in the casing 10; and an end cap 10 installed in the casing 10.

The casing 10 constitutes an external shape of the insulin injection device according to the invention and houses the syringe 20, the cartridge 30, the insulin injection unit 40, the Peltier module 50, the suction unit 60, and the like inside. The casing 10 is configured with a syringe cover 11 and a top cover 12 installed on an upper end of the syringe cover 11.

The top cover 12 of the casing 10 is provided with an on/off switch 12a, and when the on/off switch 12a is turned on, the insulin injection device is activated to be switched to a ready-to-operate state.

The syringe 20 is tubular with a hollow interior, with a guide 21 installed at a lower end, and with a cartridge 30 housed inside syringe.

The guide 21 has the lower end of the guide in close contact with the skin S of the user (patient) during the insulin injection, and the diameter of the guide increases as the guide goes down to the lower side. Such a guide 21 can be made of transparent, translucent or opaque material, but it is preferable to made of opaque material in that a needle 31a of the cartridge 30 inside the guide 21 needs not be visible to eliminate fear of the needle 31a.

Therefore, at the guide 21, the upper end portion is installed in the lower end of the syringe 20, and the lower side portion of the space formed between the syringe 20 and the cartridge 30 is sealed. As a result, when the suction unit 60 sucks the air between the syringe 20 and the cartridge 30, external air is prevented from flowing into the interior of the syringe 20 through the lower side of the syringe 20.

Herein, the guide 21 is fixed to the end of the syringe 20 in a shape whose diameter increases as the guide goes down and the lower end is in close contact with the skin S, and from the time the air between the syringe 20 and the cartridge 30 is sucked and the skin S is pulled up to the time the insulin is injected into the skin through the needle 31a, the cartridge 30 maintains the close contact state without moving.

The cartridge 30 is housed inside the syringe 20 and is provided with a piston 32 on an upper side and the needle 31a surrounded by the guide 21 at the lower side. Thus, when the piston 32 is pressed, the insulin stored inside is injected into the skin S through the needle 31a.

Supplementally, the cartridge 30 is configured with a cylinder 31, a piston 32 installed in the cylinder 31, an oscillator 33 installed in the piston 32, and an oscillator cover 34 installed on the oscillator 33.

The cylinder 31 has the insulin stored inside and is provided with a needle 31a at the lower end for injecting the insulin into the skin S.

The piston 32 is installed in and out of the cylinder 31 to be able to move forward and backward, so that, when piston moves forward inside the cylinder 31, the insulin is injected into the skin S through the needle 31a.

The oscillator 33 is installed at the lower end of the piston 32 and mixes the insulin by vibrating it.

The oscillator cover 34 surrounds the outer side surface of the oscillator 33 and moves forward with an oscillator cover when the piston 32 moves forward to pressurize the insulin inside the cylinder 31.

On the other hand, the cartridge 30 is not limited to the structure described above, and various commercially available cartridges of various forms and structures are presented, but any cartridge can be used as long as the insulin is injected inside the skin S through the needle 31a under pressure by the insulin injection unit 40.

The the insulin injection unit 40 pushes the piston 32 of the cartridge 30 to inject the insulin into the skin S through the needle 31a. Supplementally, the insulin injection unit 40 allows the needle 31a not to directly move forward so that the needle 31a punctures the skin S, but in a stat of the guide 21 being in contact with the skin S, the needle 31a is stopped at its place. The skin S is lifted up by the operation of the suction unit 60, and the skin S is punctured by the needle 31a.
In other words, when the air between the syringe 20 and the cartridge 30 is sucked by the suction unit 60 and the pressure of the interior of the syringe 20 drops down near vacuum, the skin is lifted upward, and the skin S is punctured by the needle in the process of being lifted up in this manner. Therefore, the insulin injection unit 40 pushes the piston 32 of the cartridge 30 when the pressure of the air being sucked by the suction unit 60 is maintained within a certain range for a certain period of time. In other words, the air between the syringe 20 and the cartridge 30 is sucked by a vacuum pump 62 of the suction unit 60 to be described below. At this time, if the pressure value measured by the pressure sensor 63 to be described below is maintained within a certain range for a certain period of time, the insulin injection unit 40 is automatically operated to inject the insulin into the skin S.

The the insulin injection unit 40 as described above is configured with a drive motor 41, a screw shaft 42 connected to the motor shaft of the drive motor 41, and a piston pressing block 43 connected to the screw shaft 42.

The drive motor 41 rotates the motor shaft when power is supplied.

The screw shaft 42 is inserted inside the syringe 20 and rotates together with the motor shaft of the drive motor 41. The screw shaft 42 has a spiral formed on an outer circumference face.

The piston pressing block 43 has an inner circumference face spirally coupled to this screw shaft 42 on the inner circumference face and moves forward and backward in a straight line in the syringe 20 as the screw shaft 42 rotates,

If the piston pressing block 43 moves forward, the piston 32 of the cartridge 30 is pressurized so that the piston 32 pushes drug toward the needle 31a.

Supplementally, a pair of rails 22 are formed inside the syringe 20, and ribs 43a are formed protruding from an outer surface of the piston pressing block 43, which are interposed between the rails 22, and when the screw shaft 42 rotates, the ribs 43a move forward in a straight line along the rail 22 and the piston pressing block 43 moves forward., so that the piston 32 of the cartridge 30 is pressurized.

The Peltier module 50 supplies the cold or warm air to the syringe 20 to maintain the temperature of the interior of the syringe 20 housing the cartridge 30 within a set temperature range. In order for the insulin remaining inside the cartridge 30 after a certain amount of the insulin after injection to be stored safely without deterioration of the insulin, it needs to be stored below or above a certain temperature according to the type of the insulin. In order to maintain an appropriate storage temperature, the invention uses the Peltier module 50 matching the temperature of the interior of the syringe 20 within a desired temperature range.

Such a Peltier module 50 is configured with a temperature sensor 51, a Peltier element 52 generating cold or warm air, a conducting plate 53 facing side surface of the Peltier element 52, a heat sink plate 54 facing the other side surface of the Peltier element 52, and an blower fan 55 providing airflow to the heat sink plate 54. The temperature sensor 51 is installed in the syringe 20 to measure the temperature of the syringe 20, and the measured temperature value is transmitted to the control unit 70 for display. The Peltier element 52 generates the cold or warm air according to the value measured by the temperature sensor 51. In other words, when the temperature of the interior of the syringe 20 is below a proper storage temperature of the insulin, the warm air is generated from the Peltier element 52 according to a command of the control unit 70. When the temperature exceeds the proper storage temperature of the insulin, the cool air is generated from the Peltier element 52 by the command of the control unit 70.

The conducting plate 53, which connects the syringe 20 and the Peltier element 52, is made of metal with excellent thermal conductivity, connected to the Peltier element 52 at one side and connected to the syringe 20 at the other side, and is supplied with the cold or warm air from the Peltier element 52 and transferred to the syringe 20. At this time, it is preferable that the syringe 20 is configured with metal having an excellent thermal conductivity so that the cold or warm air can be transferred well.

The heat sink plate 54 dissipates the heat generated from the Peltier element 52.

The blower fan 55 facilitates the dissipation of the heat performed by the heat sink plate 54.

The suction unit 60 sucks the air in the space formed between the cartridge 30 and the syringe 20 when the guide 21 is in contact with the skin S to form a vacuum pressure in that space, so that the skin S is pulled up. And the skin S thus pulled up is punctured by the needle 31a. Supplementally, the suction unit 60 causes the skin S to be lifted upward in the guide 21, and the skin S thus lifted up is automatically punctured by the needle 31a inside the guide 21.

The suction unit 60 is configured with a suction tube 61, a vacuum pump 62 connected to the suction tube 61 to suck air, and a pressure sensor 63 measuring the pressure of air sucked by the vacuum pump 62.

The suction tube 61 once penetrates the syringe 20 and is connected to the space between the cartridge 30 and the syringe 20.

The vacuum pump 62 is connected to the other side end of the suction tube 61 and draws air in the space between the cartridge 30 and the syringe 20. The air pressure of a vacuum or near-vacuum is formed between the cartridge 30 and the syringe 20, and due to this pressure change, the skin S is pulled up toward the needle 31a. The pressure sensor 63 is installed on the conduit of the suction tube 61 or at the end of the suction tube 61 to measure the pressure of the air being sucked by the vacuum pump 62. The pressure value measured in this manner is transmitted to the control unit 70 for display. The control unit 70 is installed on a top cover 12 of the casing 10 and is electrically connected to the insulin injection unit 40, the Peltier module 50 and the suction unit 60 to control so as to allow these components to be operated or not operated

The control unit 70 is supplied with information about the insulin injection amount, the number of times of the insulin injection, the pressure inside the syringe 20, the temperature of the syringe 20, and the like by the insulin injection unit 40, the suction unit 60, and the temperature sensor 51, and the like for display and also transmits the information to a smartphone connected through wired or wireless communication network. The information is transmitted to a smartphone connected through a wired or wireless communication network. Therefore, the afore-mentioned information can be confirmed through the smartphone.
On the other hand, when the temperature of the syringe 20 is outside the set temperature range, the control unit 70 can be commanded so as to operate the Peltier module 50 through the smartphone. That, when it is confirmed through the smartphone that the temperature of the syringe 20 is not appropriate for storing the insulin, the user transmits a signal through the smartphone to the control unit 70 so as to operate the Peltier module 50. The end cap 80 is installed on an upper end of the casing 10, that is, an top cover 12, and a number of air discharge holes 81 discharging air sucked by the suction unit 60 to the outside are formed. The air discharge holes 81 are formed at regular intervals along the edge of the end cap 80 so as to form a circular shape. On the other hand, a battery 90 capable of storing electricity is installed inside the casing 10 to supply electricity to the suction unit 60, the insulin injection unit 40, the Peltier module 50, the control unit 70, and the like., so that the insulin injection device is enabled to be used wireless. A charging hole 82 is formed in the center of the end cap 80 which allows electricity to be supplied and stored to the battery 90. Hereinafter, a behavior of the operation of the thermostatically storable smart insulin injection device according to the invention configured as described above will be described in brief.
First, when the on/off switch 12a is operated and turned on, the suction unit 60 begins to operate, and in this state the guide 21 is allowed to in contact with the skin S of the user (patient) . In a state of the guide 21 being in contact with the skin S, the air between the syringe 20 and the cartridge 30 is sucked by the suction unit 60, and the air is discharged outside through the air discharge hole 81. While these processes continue, the pressure in the space between the syringe 20 and the cartridge 30 decreases, and the skin S is lifted upward.

As the skin S is lifted up, the needle 31a is automatically punctured into the skin S.

After the needle 31a is punctured into the skin S, when the pressure value measured by the pressure sensor 63 remains within a certain range for a certain period of time, the insulin injection unit 40 is operated and the piston pressing block 43 pushes the piston 32 of the cartridge 30 to inject the insulin into the skin S.

This state of the insulin being injected into the skin S is maintained for a certain period of time (for, 10 seconds) . The reason for maintaining the insulin in this manner is to prevent the insulin loss due to the insulin backflow in the early stage of injection while ensuring sufficient time for the insulin injection.
After the insulin injection is maintained for a certain period of time, the electricity supplied to the suction unit 60 and the insulin injection unit 40 is cut off, so that the vacuum state is released, and the insulin is no longer injected into the skin S. If the insulin remains inside the cartridge 30 after the insulin injection is completed, the Peltier module 50 is allowed to be operated, so that the temperature of the interior of the syringe 20 is set to an appropriate temperature at which the insulin does not deteriorate.

### INDUSTRIAL AVAILABILITY

The present invention relating to a smart insulin injection device capable of storing insulin at a constant temperature can be widely used in bio industries and industries related to medical devices.

## Claims

1. A thermostatically-storable smart insulin injection device comprising:
a casing;
a syringe installed inside the casing and with a guide at a lower end to be in contact with skin during insulin injection;
a cartridge housed inside the syringe in a state insulin of being stored, and with a piston installed at an upper side and with a needle surrounded by the guide at the lower side;
an insulin injection unit pushing the piston of the cartridge to inject the insulin into skin through the needle; and
a Peltier module supplying cold or warm air to the syringe to maintain the temperature of the interior of the syringe housed in a cartridge within a set temperature range.

2. The thermostatically-storable smart insulin injection device according to claim 1, wherein the insulin injection unit includes:
a drive motor;
a screw shaft connected to a motor shaft of the drive motor and rotating with the motor shaft; and
a piston pressing block connected to the screw shaft and pushing the piston of the cartridge by moving forward in the syringe.

3. The thermostatically-storable smart insulin injection device according to claim 1, wherein the Peltier module is configured with:
a temperature sensor measuring the temperature of the syringe;
a Peltier element generating the cold or warm air according to a value measured by the temperature sensor;
a conducting plate facing one side surface of the Peltier element and being in contact with the syringe to transfer the cold or warm air;
a heat sink plate facing the other side surface of the Peltier element; and
a blower fan providing airflow to the heat sink plate.

4. The thermostatically-storable smart insulin injection device according to claim 1, further comprising a suction unit which, when the guide is contact with the skin, sucks the air between the cartridge and the syringe so that the skin is pulled up, to allow the skin to be automatically punctured by the needle.

5. The thermostatically-storable smart insulin injection device according to claim 4, wherein the suction unit is configured with:
a suction tube with one side end being connected to a space between the cartridge and the syringe;
a vacuum pump being connected to the other side end of the suction tube to suck air in the space between the cartridge and the syringe; and
a pressure sensor installed on the conduit of the suction tube or at the end of the suction tube to measure the pressure of the sucked air.

6. The thermostatically-storable smart insulin injection device according to claim 4,
wherein a control unit is electrically connected to the insulin injection unit, the Peltier module, and the suction unit, and
wherein the control unit displays information about the insulin injection amount, the number of times of insulin injection, and the temperature of the syringe, and transmits the information to a smartphone connected through a communication network.

7. The thermostatically-storable smart insulin injection device according to claim 6, wherein commanding the control unit so as to operate the Peltier module through the smart phone when the temperature of the syringe is outside the set temperature range.

8. The thermostatically-storable smart insulin injection device according to claim 4, wherein an end cap with a plurality of air discharge holes discharging air sucked by the suction unit to the outside formed along the edge is installed at an upper end of the casing.

9. The thermostatically-storable smart insulin injection device according to claim 8, wherein a battery supplying electricity to the suction unit and the insulin injection unit is installed inside the casing, and a charging hole supplying electricity to the battery to be stored is formed in the end cap.

10. A auto-injectable smart insulin injection device wherein a cartridge is configured with:
a cylinder provided with a needle at the lower end and insulin stored therein;
a piston installed inside and outside the cylinder and able to be moved back and forth;
an oscillator installed at a lower end of the piston to oscillate the insulin; and
an oscillator cover surrounding an outer surface of the oscillator and moves forward with an oscillator cover when the piston moves forward to pressurize the insulin.
